# FASCICULE DE BREVET EUROPEEN

(11) **EP 0 778 885 B1**
(45) Date de publication et mention de la délivrance du brevet: **08.11.2006**
(21) Numéro de dépôt: 95929915.7
(22) Date de dépôt: 28.08.1995
(51) Int. Cl.: C12N 1/20, A23C 9/123, C12N 9/38, C12P 19/14

(54) **PREPARATION DE PRODUITS FERMENTES PAR STREPTOCOCCUS THERMOPHILUS, ENRICHIS EN GALACTO-OLIGOSACCHARIDES ET EN BETA-GALACTOSIDASE**
HERSTELLUNG VON DURCH STREPTOCOCCUS THERMOPHILUS FERMENTIERTEN PRODUKTEN MIT HOHEM GEHALT VON GALACTO-OLIGOSACCHARIDEN UND BETA-GALACTOSIDASE
PREPARATION OF GALACTO-OLIGOSACCHARIDE AND BETA-GALACTOSIDASE ENRICHED PRODUCTS FERMENTED USING STREPTOCOCCUS THERMOPHILUS

(30) Priorité: 31.08.1994 FR 9410468
(43) Date de publication de la demande: 18.06.1997
(73) Titulaire: COMPAGNIE GERVAIS-DANONE, F-92302 Levallois Perret (FR)
(72) Inventeur: BLAREAU, Jean-Pierre, F-59114 Steenvoorde (FR); LECROIX, Francis, F-59270 Godewaersvelde (FR); MAERTEN, Bernard, F-59190 Hazebrouck (FR); PRONNIER, Paul, F-19100 Brive-la-Gaillarde (FR)
(74) Mandataire: Vialle-Presles, Marie José
(86) Numéro de dépôt international: PCT/FR1995/001126
(87) Numéro de publication internationale: WO 1996/006924

(56) Documents cités:
- EP-A- 0 323 201
- EP-A- 0 538 646
- EP-B- 0 529 414
- JOURNAL OF DAIRY RESEARCH, vol. 56, 1989 G.B., pages 785-792, B-S. CHANG & R.R. MAHONEY 'Factors affecting the thermostability of beta-galactosidase (Streptococcus salivarius subsp. thermophilus) in milk: a quantitative study.'
- APPLIED MICROBIOLOGY & BIOTECHNOLOGY., vol. 34, no. 4, Janvier 1991 SPRINGER-VERLAG, pages 495-501, J.B. SMART 'Transferase reactions of the beta-galactosidase from Streptococcus thermophilus.' cité dans la demande

## Description

La présente Invention est relative à un procédé de préparation de produits lactés fermentés convenant tout particulièrement à l'alimentation des nourrissons.

On sait que les produits lactés fermentés du type yoghourt présentent un grand intérêt pour l'alimentation infantile. En effet, les bactéries lactiques, traditionnellement utilisées pour la fabrication de ces produits présentent une activité β-galactosidase relativement élevée, ce qui améliore la digestibilité du lactose, et en conséquence, la tolérance de l'aliment.

La β-galactosidase est responsable de l'hydrolyse du lactose en glucose et galactose. Des études sur l'activité de préparations de β-galactosidase ont montré qu'à partir d'une certaine concentration de lactose, cette enzyme catalyse également, parallèlement à l'hydrolyse du lactose qui aboutit à la formation de glucose et galactose, une réaction de transgalactosylation qui aboutit à la production d'une famille d'oligosaccharides (di- à hexa-saccharides) dénommés galacto-oligosaccharides [voir par exemple, SMART, Appl. Microbiol. Biotechnol., 34, 495-501 (1991)].

Or, il a été démontré que les galacto-oligosaccharides constituent un facteur stimulant la croissance des Bifidobactéries, et permettraient donc un enrichissement de la flore intestinale en Bifidobactéries au dépens d'autres microorganismes, en particulier des bactéries pathogènes [TANAKA et al., Bifidobacteria Microflora, Vol. 2 (1), 17-24, (1983)].

Il a été proposé d'utiliser des β-galactosidases de différentes origines pour préparer des galacto-oligosaccharides, par exemple pour obtenir des produits laitiers à teneur élevée en galacto-oligosaccharides.

Deux catégories de procédés tendant à ce but ont été décrites :
1) La première catégorie met en oeuvre le traitement d'un substrat contenant du lactose, tel que le lait, par des préparations de β-galactosidase purifiée à partir de différents microorganismes.
   On connaît ainsi par la Demande de Brevet Européen N° 323 201 au nom de YAKULT HONSHA KK, un procédé qui consiste à traiter un lait animal par une préparation de β-galactosidase extraite de *Streptococcus thermophilus* ou de *Lactobacillus bulgaricus,* dans le but de transformer au moins 15% du lactose contenu dans ce lait, en galacto-oligosaccharides.
   Il a également été proposé [Z. MOZAFFAR et al., Journal of Food Science, 50, p. 1602-1606, (1985)] de produire des galacto-oligosaccharides à partir du lait en utilisant une β-galactosidase extraite de *Bacillus circulans.*
   Ces procédés présentent l'inconvénient de nécessiter la purification préalable de la β-galactosidase, et posent les problèmes de stabilisation de l'activité enzymatique qui existent, de manière générale avec toute préparation d'enzyme purifiée.
2) Une deuxième catégorie de procédés propose l'utilisation de bactéries présentant une activité β-galactosidase, sans extraction préalable de l'enzyme. Toutefois, se pose alors le problème de rendre la β-galactosidase, qui est intracytoplasmique, accessible à son substrat.

Pour cela, on doit procèder soit à la lyse des bactéries, soit au moins à leur perméabilisation, par exemple par un solvant organique ou un tensio-actif. La perméabilisation, bien que moins drastique que la lyse, est toutefois néfaste pour la viabilité des bactéries, et affecte également l'activité β-galactosidase ; il a été proposé en conséquence de traiter les bactéries perméabilisées, afin de prolonger leur viabilité et de protéger l'activité β-galactosidase.

Des traitements par le glycérol ou le sorbitol ont par exemple été préconisés dans ce but. Un autre type de traitement, tel que celui décrit dans la Demande de Brevet Japonais au nom de YAKULT HONSHA KK, publiée le 28 Juin 1991 sous le numéro 3 151 875, met en oeuvre un procédé où des bactéries perméabilisées sont traitées par des galacto-oligosaccharides afin de protèger le site actif de la β-galactosidase. Ceci permet de retarder l'inactivation de cette enzyme au cours de la conservation des bactéries, et pendant la réaction de formation des galacto-oligosaccharides.

Quel que soit celui de ces deux types de procédé qui est mis en oeuvre, même si une activité β-galactosidase est encore présente dans le produit après la réaction de transgalactosylation, cette activité n'est pas conservée dans le produit fini prêt à la consommation. En effet, ni l'enzyme purifiée ni celle présente dans les bactéries perméabilisées, ne résiste aux différents traitements aboutissant à l'obtention du produit fini. A fortiori, l'activité β-galactosidase ne résiste pas à l'action des sucs digestifs après ingestion par le consommateur.

Bien qu'il soit souhaitable, en particulier pour l'alimentation de l'enfant et du nourisson, de disposer de produits laitiers enrichis à la fois en β-galactosidase, et en galacto-oligosaccharides, il n'avait, jusqu'à présent pas été proposé d'utiliser un même microorganisme pour parvenir à ce double objectif.

La présente Invention a pour but la préparation de produits laitiers enrichis à la fois en β-galactosidase, et en galacto-oligosaccharides.

Cette préparation fait intervenir un microorganisme possédant une activité β-galactosidase qui est dans un premier temps utilisée pour la production de galacto-oligosaccharides, dans des conditions où le microorganisme demeure intact, ce qui permet dans un deuxième temps de protéger l'activité β-galactosidase, d'abord au cours des diverses manipulations aboutissant au produit prêt à la consommation, et ensuite, après ingestion par le consommateur, de l'action des sucs digestifs jusqu'au niveau intestinal.

Les Inventeurs ont choisi d'utiliser la bactérie *Streptococcus thermophilus* dans ce but.

*Streptococcus thermophilus* est une bactérie Gram-positive, aérobie-anaérobie facultative. Elle réalise une fermentation homolactique.

Cette bactérie présente différentes caractéristiques permettant son incorporation directe dans des produits pouvant être conservés à l'état frais, aussi bien que dans des produits destinés à supporter une grande variété de traitements (déshydratation par exemple), et qui peuvent être administrés en particulier à des nourrissons.

En particulier, *Streptococcus thermophilus* :
- ne produit que l'acide lactique L(+), et en outre en quantité faible, contrairement aux microorganismes du type *Lactobacillus,* qui produisent de l'acide lactique D(-) non métabolisable par le nourrisson ;
- est très résistante à la dessication à température élevée, qui est pratiquée pour l'obtention des laits en poudre ;
- ne possède qu'une activité protéolytique très faible, ce qui limite les risques de formation de composés ayant un goût désagréable au cours du stockage des produits finis.

*S. thermophilus* possède une β-galactosidase intracellulaire dans des conditions normales de culture, ce qui pose les problèmes d'accessibilité au substrat qui ont été mentionnés ci-dessus.

MOLOTOV et al. [Biotekhnologiya, 2, 33-37, (1991)] rapportent, contrairement aux observations faites par la majorité des auteurs, l'existence dans certaines conditions de culture d'une activité β-galactosidase excrétée par les bactéries. Cette excrétion ne représente toutefois qu'un phénomène marginal, qui a été observé dans des cultures agées, ou dans des cultures effectuées en présence de glucose à la place du lactose.

Or, pour envisager l'utilisation de *S*. *thermophilus* pour la préparation de produits laitiers fermentés enrichis en β-galactosidase et en galacto-oligosaccharides, il faut obtenir une bonne croissance bactérienne, afin d'une part de permettre la fermentation du substrat et d'autre part d'assurer la présence au cours de la fermentation, d'une activité β-galactosidase suffisante pour permettre d'envisager un enrichissement conséquent du produit fermenté en galacto-oligosaccharides. Les conditions dans lesquels MOLOTOV et al. ont fait leurs observations sont peu favorables à une bonne croissance des bactéries et à l'obtention d'une activité β-galactosidase élevée, et en outre ne sont pas propices à la réaction de transgalactosylation qui permet d'obtenir les galacto-oligosaccharides.

Or, les Inventeurs ont maintenant découvert qu'il était possible de cultiver certaines souches de *S*. *thermophilus,* sur un milieu contenant du lactose, en particulier un milieu à base d'un concentré de lait, de manière à obtenir une fermentation du milieu s'accompagnant d'une production élevée de galacto-oligosaccharides, représentant entre 2 et 6 % du lactose initialement présent dans le milieu de culture, et obtenue en conservant les cellules intactes.

La présente Invention a pour objet un procédé de préparation d'un produit fermenté enrichi en galacto-oligosaccharides, lequel procédé est caractérisé en ce qu'il comprend une étape au cours de laquelle l'on met en culture une souche de *S*. *thermophilus* dans un milieu dont la concentration en poids de matière sèche est d'au moins 15% , et qui comprend outre les nutriments nécessaires à la croissance de *S*. *thermophilus,* au moins 0,1% (en poids sur la matière sèche) d'un hydrolysat de protéines de lait ayant un degré d'hydrolyse compris entre 15 et 50%, et au moins 20% (en poids sur la matière sèche) de lactose.

Les conditions de culture habituelles de *S*. *thermophilus,* et les nutriments nécessaires à sa croissance sont bien connus de l'homme du métier : il est par exemple usuel de cultiver cette bactérie sur milieu M₁₇ ou sur lait écrémé reconstitué à 10% de matière sèche, supplémenté de 0,1% d'extrait de levure.

Selon un mode de mise en oeuvre préféré du procédé conforme à l'Invention, la concentration en matière sèche du milieu est comprise entre 30 et 50% et ledit milieu comprend entre 0,2 et 3% d'un hydrolysat de protéines de lait, et entre 25 et 60% en poids sur la matière sèche, de lactose.

On entend par "hydrolysat de protéines de lait" un hydrolysat de caséine, un hydrolysat de rétentat d'ultrafiltration de lait, ou un hydrolysat de protéines complètes de lait. On peut, si on le souhaite, utiliser une fraction peptidique purifiée produite par ultrafiltration d'un des hydrolysats mentionné ci-dessus. Dans ce cas, on choisira une fraction comprenant des peptides de poids moléculaire moyen inférieur à 5000 Da, de préférence inférieur à 2000 Da.

Selon un autre mode de mise en oeuvre préféré de la présente Invention, ledit hydrolysat de protéine de lait a un degré d'hydrolyse compris entre 15 et 50%, de préférence supérieur à 20%.

Le degré d'hydrolyse (DH) est défini comme étant le pourcentage d'azote aminé par rapport à l'azote total.

Préférentiellement, cet hydrolysat est obtenu par hydrolyse enzymatique de la préparation de protéines de lait. De nombreuses protéases, par exemple la trypsine (EC 3.4.21.4), la chymotrypsine (EC 3.4.21.1), ou des mélanges de protéases tels que la catalase, la pancréatine, etc ... peuvent convenir à l'obtention de cet hydrolysat. Les conditions d'hydrolyse seront choisies pour atteindre le DH indiqué ci-dessus.

La présente Invention a également pour objet des milieux de culture permettant la mise en oeuvre du procédé conforme à l'Invention.

Pour mettre en oeuvre le procédé conforme à l'Invention, on peut partir d'une solution à base de lait à laquelle on ajoute, outre les constituants permettant la mise en oeuvre du procédé de l'Invention, les ingrédients nécessaires à la réalisation du produit prêt à consommer que l'on souhaite obtenir. Si par exemple on souhaite obtenir un aliment lacté pour nourrissons, on ajoutera du lactose, des malto-dextrines, des minéraux, des vitamines, des matières grasses, les ingrédients permettant de reconstituer la composition du lait maternel. Si on le souhaite, les matières grasses sont incorporées, puis homogénéisées avec la solution de manière à obtenir une émulsion stable.

La solution est alors concentrée dans une fourchette d'extrait sec située entre 15 et 50%, de préférence entre 30 et 45 %, de matière sèche, avant d'être refroidie entre 35 et 55°C, de préférence entre 37 et 45°C et ensemencée avec une culture de *Streptococcus thermophilus* contenant 10⁷ à 10⁹ bactéries/ml. La température optimale de culture est comprise entre 40 et 45°C.

Le choix d'une souche de *S. Thermophilus* permettant d'obtenir les meilleurs résultats sera effectué par sélection préalable par culture sur un milieu conforme à l'Invention ; l'activité β-galactosidase souhaitée dans le produit prêt à l'emploi étant supérieure à 0,15U.β-gal/ml, de préférence comprise entre 0,2 et 0,4 U.β-gal/ml, on choisira une souche présentant une activité en culture sur le milieu conforme à l'Invention supérieure à 0,9U β-gal/g au bout de 4h30 de culture. Une unité β-galactosidase correspond à une micromole d'ONPG hydrolysée par minute, à pH 7,3, et à 37°C.

A titre d'exemple, des souches de *S. thermophilus* disponibles dans le commerce et qui conviennent particulièrement bien à la mise en oeuvre du procédé de l'Invention sont les souches ST 12 et ST 13 commercialisées par la société BOLL (Le Moulin d'Aulnay ; BP 64 ; Saint Germain les-Arpajon 91292 Arpajon Cedex France).

D'autre part, des souches de *S. thermophilus* convenant également à la mise en oeuvre de l'Invention, ont été déposées auprès de la CNCM (Collection Nationale de Cultures de Microorganismes) tenue par l'Institut Pasteur, 25 rue du Docteur Roux, à Paris : la souche dénommée LFL-01 a été déposée le 25 août 1994, sous le numéro I-1470, et la souche dénommée DN-001065 a été déposée le 23 août 1995, sous le numéro I-1620.

Lors de la fermentation du substrat par S. *thermophilus* au cours de la mise en oeuvre du procédé de l'Invention, l'activité β-galactosidase se développe en parallèle avec l'acidité. La mesure du pH dans des conditions de culture et pour une composition de milieu définies permet donc de suivre si l'activité β-galactosidase désirée est obtenue. Le produit est alors refroidi entre 10 et 25°C, afin de suspendre la fermentation.

On peut inoculer en outre le milieu de fermentation avec des bifidobactéries telles que *Bifidobactérium breve, Bifidobactérium infantis, Bifidobactérium longum, Bifidobactérium bifidum.*

Ces bactéries peuvent être ajoutées en début de fermentation par *Streptococcus thermophilus*, au cours de celle-ci, ou après celle-ci.

Référence du dossier du
deposant ou du mandataire | F191/110APCT | Demande internationale n°

INDICATIONS RELATIVES A UN MICRO-ORGANISME DEPOSE

(règle 13bis du PCT)

A. Les indications ont trait au micro-organisme visé dans la description
page 8 , ligne 18 .

B. IDENTIFICATION DU DEPOT | D'autres dépôts font l'objet d'une feuille supplémentaire [X]

Nom de l'institution de dépôt
CNCM (Collection Nationale de Cultures de Microorganismes)

Adresse de l'institution de dépôt (y compris le code postal et le pays)
INSTITUT PASTEUR
25 rue du Docteur Roux, 75015 PARIS
FRANCE

Date du dépôt 25 Août 1994 (25.08.1994) | n° d'ordre I-1470

C. INDICATIONS SUPPLEMENTAIRES (le cas échéant) | Une feuille supplémentaire est jointe
pour la suite de ces renseignements [X]

En ce qui concerne les désignations dans lesquelles un brevet européen est demandé
un échantillon du micro-organisme déposé ne sera accessible, jusqu'à la publica-
tion de la mention de la délivrance du brevet européen ou jusqu'à la date à
laquelle la demande sera rejetée, retirée ou réputée retirée, que par la remise
d'un échantillon à un expert désigné par le requérant

D. ETATS DESIGNES POUR LESQUELS LES INDICATIONS SONT DONNEES
(si les indications ne sont pas données pour tous les Etats désignés)

E. INDICATIONS FOURNIES SEPAREMENT (le cas échéant)

Les indications énumérées ci-après seront fournies ultérieurement au Bureau international (spécifier la nature générale des indications
p. ex., "n° d'ordre du dépôt")

Réservé à l'office récepteur | Réservé au Bureau international

[ ] Cette feuille a été reçue en même temps que la
demande internationale 28 AOUT 1995 | [ ] Cette feuille est parvenue au Bureau international le :

Fonctionnaire autorisé
N. SAADA | Fonctionnaire autorisé

Formulaire PCT/RO/134 (juillet 1992)

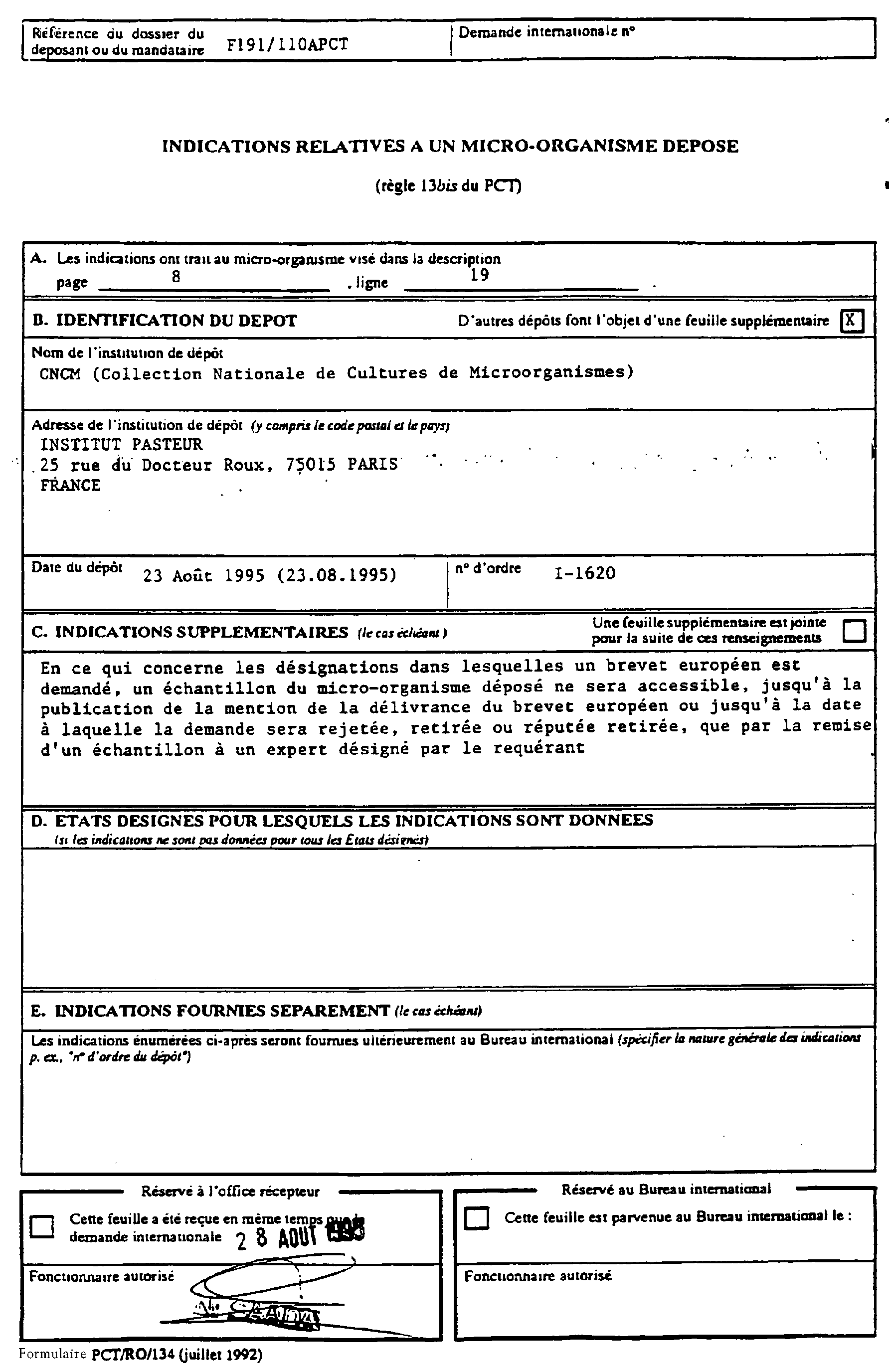
Référence du dossier du
deposant ou du mandataire F191/110APCT | Demande internationale n°

INDICATIONS RELATIVES A UN MICRO-ORGANISME DEPOSE

(règle 13bis du PCT)

A. Les indications ont trait au micro-organisme visé dans la description
page 8 , ligne 19 .

B. IDENTIFICATION DU DEPOT | D'autres dépôts font l'objet d'une feuille supplémentaire [X]

Nom de l'institution de dépôt
CNCM (Collection Nationale de Cultures de Microorganismes)

Adresse de l'institution de dépôt (y compris le code postal et le pays)
INSTITUT PASTEUR
25 rue du Docteur Roux, 75015 PARIS
FRANCE

Date du dépôt 23 Août 1995 (23.08.1995) | n° d'ordre I-1620

C. INDICATIONS SUPPLEMENTAIRES (le cas échéant) | Une feuille supplémentaire est jointe pour la suite de ces renseignements [ ]

En ce qui concerne les désignations dans lesquelles un brevet européen est
demandé, un échantillon du micro-organisme déposé ne sera accessible, jusqu'à la
publication de la mention de la délivrance du brevet européen ou jusqu'à la date
à laquelle la demande sera rejetée, retirée ou réputée retirée, que par la remise
d'un échantillon à un expert désigné par le requérant

D. ETATS DESIGNES POUR LESQUELS LES INDICATIONS SONT DONNEES
(si les indications ne sont pas données pour tous les Etats désignés)

E. INDICATIONS FOURNIES SEPAREMENT (le cas échéant)
Les indications énumérées ci-après seront fournies ultérieurement au Bureau international (spécifier la nature générale des indications
p. ex., "n° d'ordre du dépôt")

Réservé à l'office récepteur | Réservé au Bureau international
[ ] Cette feuille a été reçue en même temps que la
demande internationale 28 AOUT 199[?] | [ ] Cette feuille est parvenue au Bureau international le :
Fonctionnaire autorisé | Fonctionnaire autorisé
 |

Formulaire PCT/RO/134 (juillet 1992)

Le produit fermenté peut ensuite subir différents traitements, dont la nature varie selon le produit prêt à consommer que l'on souhaite obtenir. Il peut par exemple être additionné d'agents de texture, de saveur, de suppléments vitaminiques ou minéraux, de matières grasses, etc..., si ceux-ci n'ont pas été ajoutés préalablement à la fermentation.

Dans le cas ou l'on souhaite obtenir un produit déshydraté, on procède alors au séchage. Le séchage peut être effectué par tout procédé usuel, par exemple par atomisation ou lyophilisation

Au cours du séchage par atomisation, et bien que la β-galactosidase de *S*. *thermophilus* soit particulièrement résistante, on préférera une température d'air de sortie qui doit être inférieure à 90°C, si l'on veut conserver le maximum d'activité β-galactosidase.

La présente Invention a également pour objet un procédé de préparation d'un aliment lacté enrichi en galacto-oligosaccharides et/ou en β-galactosidase, caractérisé en ce qu'il comprend la mise en oeuvre d'un procédé conforme à l'Invention de préparation d'un produit fermenté.

Dans ce cadre, la présente Invention englobe en particulier des procédés de préparation:
- d'aliments lactés frais ;
- d'aliments lactés déshydratés ;
- d'aliments lactés reconstitués par addition d'eau aux aliments lactés déshydratés conformes à l'Invention.

Selon un mode de réalisation préféré d'un procédé de préparation conforme à l'Invention, il est mis en oeuvre pour la préparation d'un aliment lacté déshydraté possédant une activité β-galactosidase comprise entre 0,5 et 5U β-gal, et de préférence entre 1,5 et 3U β-gal, par gramme de matière sèche, et comprenant entre 0,5 et 5 g, et de préférence entre 0,8 et 2,5 g, de galacto-oligosaccharides par 100 g de matière sèche.

De préférence, ledit aliment lacté déshydraté comprend en outre un lyophilisat de bifidobactéries. Avantageusement, ces bifidobactéries sont choisies dans le groupe constitué par *Bifidobactérium breve, Bifidobactérium infantis, Bifidobactérium longum, Bifidobactérium bifidum.*

Les produits lactés obtenus par le procédé de l'Invention sont mieux tolérés que les produits lactés connus, notamment par les sujets déficients en β-galactosidase, du fait qu'ils contiennent la β-galactosidase des bactéries lactiques vivantes, qui assure une meilleure digestibilité du lactose qu'ils contiennent ; en outre, ces produits lactés, riches en galacto-oligosaccharides, favorisent le développement des bifidobactéries de la flore intestinale *in situ.* Ils sont particulièrement bien adaptés à l'alimentation infantile.

La description qui va suivre se réfère à des exemples de mise en oeuvre de procédés conformes à l'Invention, en particulier pour la préparation de produits destinés à l'alimentation infantile.

### EXEMPLE 1 : PREPARATION D'UN MILIEU DE FERMENTATION PERMETTANT LA MISE EN OEUVRE DU PROCEDE DE L'INVENTION

A un lait de vache écrémé, réchauffé à 75°C, on ajoute de la matière grasse végétale et de la lécithine. On homogénéise à la même température en deux étapes, la première sous 200 kg/cm², la seconde sous 50 kg/cm².

On ajoute une troisième partie constituée par une solution contenant les vitamines.

Le mélange final est pasteurisé à 115°C puis concentré par évaporation à 43% de matière sèche. Le concentré présente la composition suivante (Tableau I) en exprimée en grammes pour 100 grammes de matière sèche.

**TABLEAU I**

| | |
|---|---|
| Matière grasse végétale | 24 g |
| dont lécithine | 0,25 g |
| Protéines de lait (dont 80% de caséines et 20% de prot. sériques) | 12,85 g |
| Lactose | 40 g |
| Malto dextrines | 20,4 g |
| Sels minéraux | 2,6 g |
| Vitamines | 0,15 g |

### EXEMPLE 2 : CHOIX D'UNE SOUCHE DE S. THERMOPHILUS.

Différentes souches de *S. thermophilus* ont été testées.

Les souches sont d'abord cultivées sur lait écrémé stérilisé enrichi en extrait de levure (1 g/l), jusqu'à obtention d'une culture comprenant environ 10⁸ germes/ml.

Un concentré de lait infantile préparé comme décrit à l'exemple 1, est ensemencé avec cette culture à raison de 5% v/v, puis incubé à 44°C.

Les acidités sont dosées, et la fermentation est arrêtée aux environs de 110°D, ou après 6 heures de fermentation si l'acidité de 110'D n'est pas atteinte. On mesure alors l'activité β-galactosidase.

Les résultats sont indiqués dans le tableau II ci-dessous : L'acidité est exprimée en degrés Dornic (°D) : un degré Dornic correspond à 1 mg d'acide lactique/10 ml de lait ; l'activité β-galactosidase est exprimée en U β-gal/g de concentré.

**TABLEAU II**

| ACIDITE (°D) | | | | | β-gal |
|---|---|---|---|---|---|
| Souche | 2H | 3H30 | 4H30 | 6H | fin fermentation |
| STB01* | 60 | 95 | 115 | - | 0,53 |
| STB05* | 58 | 96 | 115 | - | 0,52 |
| TH3* | 59 | 93 | 110 | - | 0,42 |
| ST9* | 43 | 50 | 58 | 63 | pas caillé |
| DPch | 61 | 100 | 115 | - | 0,93 |
| ST37* | 47 | 60 | 86 | 114 | 0,55 |
| LFL-01 | 61 | 96 | 112 | - | 1 |
| DN-001065 | 43 | 53 | 63 | 92 | 1,30 |

| | | | | | |
|---|---|---|---|---|---|
| * : Souches commercialisées par la Société BOLL | | | | | |

Les souches ST12 et ST 13 (BOLL) ont été testées dans les mêmes conditions, et permettent d'obtenir respectivement 0,97 et 0,94 U β-gal/g de concentré au bout de 4 heures 30 d'incubation.
Les souches DPch, LFL-01, DN-001065, ainsi que les souches ST12 et ST 13 possèdent une activité permettant la mise en oeuvre du procédé de l'Invention.

### EXEMPLE 3 : INFLUENCE DE LA NATURE DE L'HYDROLYSAT DE PROTEINES SUR L'ACTIVITE β-GALACTOSIDASE

Pour étudier l'influence du type d'hydrolysat de protéine et du degré d'hydrolyse sur l'activité β-galactosidase, des fractions aliquotes d'un milieu de départ obtenu comme décrit à l'exemple 1, et concentré à 40%, sont additionnés de différents hydrolysats, à taux isoprotéique (ce qui correspond à 1,1 g de protéine/l de solution concentrée).

Les concentrés sont inoculés avec 5% (V/V) d'une culture de *Streptococcus Thermophilus* (souche LFL-01) sur lait enrichi en extrait de levure.

La température d'incubation est de 44°C.
L'acidité et l'activité β-galactosidase sont dosées au bout de 4 heures d'incubation. Les résultats sont reproduits dans le Tableau III ci-dessous :

**TABLEAU III**

| Type d'hydrolysat | Acidité | β-gal |
|---|---|---|
| Aucun | 84°D | 0,11U β-gal/g |
| Caséine | 121°D | 0,95U β-gal/g |
| Protéines de lactosérum | 113°D | 0,45U β-gal/g |
| Soja | 115°D | 0,58U β-gal/g |
| Viande de dinde | 111°D | 0,36U β-gal/g |
| Lactalbumine | 118°D | 0,71U β-gal/g |

### EXEMPLE 4 : INFLUENCE DU TYPE D'HYDROLYSAT (CASEINE, LAIT OU RETENTAT DE LAIT) AJOUTE A UN CONCENTRE DE LAIT INFANTILE SUR L'ACTIVITE β-GALACTOSIDASE

Les cultures de *S*. *Thermophilus* (souche LFL-01), et les dosages sont effectués comme décrit à l'Exemple 3.

Pour chaque hydrolysat, trois doses sont testées : 0,9 g, 1,5 g, et 3,6 g d'hydrolysat par kg de concentré à 40%.

Les résultats sont indiqués dans le Tableau IV ci-dessous.

**TABLEAU IV**

| TYPE D'HYDROLYSAT | DOSE (g/Kg) | 3 heures | | 4 heures | |
|---|---|---|---|---|---|
| | | Acidité (°D) | Activité β-gal | Acidité (°D) | Activité β-gal |
| Caséine | 0,9 | 77 | 0,62 | 102 | 1,15 |
| | 1,5 | 79 | 0,67 | 104 | 1,09 |
| | 3,6 | 82 | 0,67 | 103 | 1,24 |
| Rétentat de lait | 0,9 | 80 | 0,63 | 107 | 0,95 |
| | 1,5 | 85 | 0,56 | 110 | 1,15 |
| | 3,6 | 93 | 0,57 | 112 | 1,31 |
| Lait | 0,9 | 88 | 0,59 | 107 | 0,93 |
| | 1,5 | 92 | 0,67 | 114 | 0,98 |
| | 3,6 | 95 | 0,71 | 114 | 1,06 |

Les résultats sont assez proches les uns des autres ; toutefois, l'hydrolysat de caséine permet d'obtenir l'activité β-galactosidase la plus élevée pour l'ajoût protéique le plus faible, et une acidité minimale.

### EXEMPLE 5 : INFLUENCE DU DEGRE D'HYDROLYSE D'UN HYDROLYSAT DE CASEINE SUR L'ACTIVITE β-GALACTOSIDASE.

Les cultures de *S. Thermophilus* (souche LFL-01), et les dosages sont effectués comme décrit à l'Exemple 3.

Un caséinate de potassium est mis en solution dans l'eau à 100 g/l, pasteurisé à 92°C, refroidi à 50°C.

La solution est transférée dans un fermenteur, équipé d'une régulation de pH et de température. La solution est alors additionnée de 1 g/l de pancréatine 3NF (Laboratoire Industriel de Biologie, Soisy-sous-Montmorency, France). La température est régulée à 50°C, et le pH est ajusté à 7,3 à la chaux.

Des échantillons sont prélevés régulièrement, portés 20 mn à 85° pour détruire l'enzyme, puis refroidis à 5°C.

Sur ces échantillons, on procède alors au dosage de l'azote total et de l'azote aminé. Le pourcentage relatif azote aminé/azote total donne le degré d'hydrolyse de la protéine.

Pour étudier l'influence du degré d'hydrolyse, des fractions aliquotes de concentré de la formule de lait infantile du tableau I sont additionnées d'une quantité identique (en azote total) des différents échantillons d'hydrolysats correspondant à des degrés d'hydrolyse croissants.

Les résultats sont consignés dans le tableau V ci-dessous.

L'activité β-galactosidase souhaitée (>0,90 U/β-gal/g) est obtenue pour les hydrolysats de caséine dont le degré d'hydrolyse est supérieur à 22%.

**TABLEAU V**

| Degré d'hydrolyse NH2/NT | 3H de fermentation | | 4H30 de fermention | |
|---|---|---|---|---|
| | Acidité en °D | β-gal U β-gal/g | Acidité en °D | β-gal U β-gal/g |
| 9% | 55 | 0,16 | 71 | 0,24 |
| 17,6% | 82 | 0,60 | 107 | 0,70 |
| 22,1% | 85 | 0,70 | 109 | 0,96 |
| 25% | 85 | 0,77 | 110 | 0,97 |
| 29,3% | 88 | 0,86 | 110 | 1,10 |
| 32,3% | 88 | 0,86 | 110 | 1,10 |
| 34,9% | 94 | 0,83 | 108 | 1,03 |

### EXEMPLE 6 : EVOLUTION DE LA PRODUCTION DE β-GALACTOSIDASE ET DE GALACTO-OLIGOSACCHARIDES AU COURS DE LA FERMENTATION D'UN CONCENTRE DE LAIT INFANTILE

Un concentré de lait est préparé comme décrit à l'exemple 1 et ensemencé à raison de 5% v/v par *S*. *Thermophilus* (souche LFL-01), puis incubé à 44°C.

L'activité β-galactosidase (U β-gal/g de concentré), la production de galacto-oligosaccharides (g pour 100 g de concentré), et l'acidité (°D) sont mesurées en fonction du temps (en heures).

Les résultats sont résumés dans le tableau VI ci-dessous :

**TABLEAU VI**

| TEMPS | ACIDITE | OLIGOSACCHARIDES | β-gal |
|---|---|---|---|
| 0 | 38 | | |
| 1 | 42 | 0,03 | 0,06 |
| 2 | 50 | 0,07 | 0,20 |
| 3 | 77 | 0,30 | 0,62 |
| 4 | 103 | 0,40 | 1,15 |
| 4,5 | 107 | 0,42 | 1,16 |

Toutes ces valeurs évoluent en parallèle et atteignent un plateau après 4h30 de fermentation.

La solution concentrée a été séchée par pulvérisation, et a été reconstituée de façon à obtenir un produit à 14% de matière sèche.

Les valeurs de l'activité β-galactosidase et de la concentration en galacto-oligosaccharides, dans le concentré, dans la poudre, puis dans le produit reconstitué, sont indiquées dans le Tableau VII ci-dessous : l'activité β-galactosidase est exprimée en U β-gal/g pour le concentré et la poudre, et en U β-gal/ml pour le produit reconstitué ; la concentration en galacto-oligosaccharides est exprimée en g pour 100 g pour le concentré et la poudre, en g/l pour le produit reconstitué.

**TABLEAU VII**

| | CONCENTRE | POUDRE | PRODUIT RECONSTITUE |
|---|---|---|---|
| β-gal | 1,16 | 2,40 | 0,33U |
| Oligosaccharides | 0,42 g%g | 0,90 g%g | 1,26 g/l |

### EXEMPLE 7 : OBTENTION DE PRODUITS PRETS A LA CONSOMMATION :

A une solution concentrée préparée comme décrit à l'Exemple 1, on inocule 5% en volume d'une culture contenant environ 10⁸ germes/ml de *Streptococcus Thermophilus* LFL-01.

On fait fermenter la solution concentrée durant environ 5 heures 30 à 44°C, jusqu'à une acidité de 106°D et une activité β-galactosidase de 1U β-gal/g de concentré.

On refroidit alors à 20°C la solution concentrée fermentée.

La préparation est ensuite séchée par pulvérisation, à une température d'entrée de tour de 165°C et une température de sortie de tour inférieure à 90°C. On obtient une poudre qui présente une activité β-galactosidase de 2,4 U/g de poudre, une teneur en eau résiduelle de environ 2 à 3% et qui peut se conserver 13 mois à 20°C en perdant au maximum 10 à 15% de son taux de β-galactosidase. Cette poudre permet, à raison de 140 g de poudre pour environ 900 ml d'eau, de reconstituer un lait acidifié, destiné en particulier aux nourrissons.

## Revendications

1. Procédé de préparation d'un produit fermenté enrichi en galacto-oligosaccharides et en β-galactosidase, lequel procédé est **caractérisé en ce qu'**il comprend une étape au cours de laquelle l'on met en culture une souche de *Streptococcus thermophilus* dans un milieu dont la concentration en matière sèche est d'au moins 15% (en poids), et qui comprend, outre les nutriments nécessaires à la croissance de ladite bactérie, au moins 0,1% (en poids sur la matière sèche) d'un hydrolysat de protéines de lait ayant un degré d'hydrolyse compris entre 15 et 50%, et au moins 20% (en poids sur la matière sèche) de lactose.

2. Procédé selon la revendication 1, **caractérisé en ce que** la concentration en matière sèche du milieu est comprise entre 30 et 50% (en poids) et **en ce que** ledit milieu comprend entre 0,2 et 3% (en poids sur la matière sèche) d'hydrolysat de protéines de lait, et entre 25 et 60% (en poids sur la matière sèche) de lactose.

3. Procédé selon la revendication 1, **caractérisé en ce que** l'hydrolysat de protéines de lait a un degré d'hydrolyse supérieur à 20%.

4. Procédé selon l'une quelconque des revendications 1 à 3, **caractérisé en ce que** le milieu est ensemencé avec une souche de *S. thermophilus* présentant une activité en culture supérieure à 0,9 U β-gal/g au bout de 4h30 de culture sur un milieu tel que défini dans la revendication 3.

5. Procédé selon la revendication 4, **caractérisé en ce que** ladite souche de *S*. *thermophilus* est choisie dans le groupe constitué par :
- la souche LFL-01, déposée le 25 août 1994 auprès de CNCM, sous le numéro 1-1470 ;
- la souche DN-001065, déposée le 23 août 1995 auprès de la CNCM, sous le numéro I-1620.

6. Procédé selon l'une quelconque des revendications 1 à 5, **caractérisé en ce que** le milieu est en outre inoculé avec des bifidobactéries.

7. Procédé selon la revendication 6, **caractérisé en ce que** lesdites bifidobactéries sont choisies dans le groupe constitué par *Bifidobacterium breve, Bifidobacterium infantis, Bifidobacterium longum, Bifidobacterium bifidum.*

8. Procédé selon l'une quelconque des revendications 1 à 7, **caractérisé en ce que**, à l'issue de la fermentation, on procède au séchage du produit fermenté.

9. Procédé selon la revendication 8, **caractérisé en ce que** le séchage est effectué par atomisation.

10. Procédé selon la revendication 9, **caractérisé en ce que**, au cours du séchage par atomisation, la température d'air de sortie est inférieure à 90°C.

11. Procédé selon la revendication 8, **caractérisé en ce que** le séchage est effectué par lyophilisation.

12. Milieu de culture tel que défini dans l'une quelconque des revendications 1 à 3.

13. Procédé de préparation d'un aliment fermenté enrichi en galacto-oligosaccharides, **caractérisé en ce qu'**il comprend la mise en oeuvre d'un procédé selon l'une quelconque des revendications 1 à 11.

14. Procédé selon la revendication 13, **caractérisé en ce qu'**il est mis en oeuvre pour la préparation d'un aliment lacté frais.

15. Procédé selon la revendication 14, **caractérisé en ce qu'**il est mis en oeuvre pour la préparation d'un aliment lacté déshydraté possédant une activité β-galactosidase comprise entre 0,5 et 5 U β-gal par gramme de matière sèche, et comprenant entre 0,5 et 5 g de galacto-oligosaccharides pour 100 g de matière sèche.

16. Procédé selon la revendication 15, **caractérisé en ce qu'**il est mis en oeuvre pour la préparation d'un aliment lacté déshydraté possédant une activité β-galactosidase comprise entre 1,5 et 3 U β-gal par gramme de matière sèche, et comprenant entre 0,8 et 2,5 g de galacto-oligosaccharides pour 100 g de matière sèche.

17. Procédé selon l'une quelconque des revendications 15 ou 16, **caractérisé en ce que** ledit aliment lacté déshydraté comprend en outre un lyophilisat de bifidobactéries.

18. Procédé selon la revendication 17, **caractérisé en ce que** lesdites bifidobactéries sont choisies dans le groupe constitué par *Bifidobacterium breve, Bifidobacterium infantis, Bifidobacterium longum, Bifidobacterium bifidum.*

19. Procédé selon une quelconque des revendications 13 à 18, **caractérisé en ce que** ledit aliment fermenté est destiné à l'alimentation infantile.

## Claims

1. Process for preparing a fermented product enriched in galacto-oligosaccharides and β-galactosidase, the process being **characterised in that** it comprises a step in which a strain of *Streptococcus thermophilus* is cultured in a medium having a concentration of dry matter of at least 15% (by weight), and which comprises, in addition to the nutrients required for the growth of said bacteria, at least 0.1% (by weight, based on dry matter) of a milk protein hydrolysate having a degree of hydrolysis of between 15 and 50% and at least 20% (by weight, based on dry matter) of lactose.

2. Process according to claim 1, **characterised in that** the concentration of dry matter of the medium is between 30 and 50% (by weight) and **in that** said medium comprises between 0.2 and 3% (by weight, based on dry matter) of milk protein hydrolysate, and between 25 and 60% (by weight, based on dry matter) of lactose.

3. Process according to claim 1, **characterised in that** the milk protein hydrolysate has a degree of hydrolysis greater than 20%.

4. Process according to any one of claims 1 to 3, **characterised in that** the medium is seeded with a strain of *S*. *thermophilus* having a culture activity greater than 0.9 U β-gal/g after 4h30 of cultivation on a medium as defined in claim 3.

5. Process according to claim 4, **characterised in that** said strain of *S*. *thermophilus* is selected from among:
- the strain LFL-01 deposited on 25 August 1994 at the CNCM under number I-1470;
- the strain DN-001065, deposited on 23 August 1995 at the CNCM, under the number I-1620.

6. Process according to any one of claims 1 to 5, **characterised in that** the medium is further inoculated with bifidobacteria.

7. Process according to claim 6, **characterised in that** said bifidobacteria are selected from among *Bifidobacterium breve, Bifidobacterium infantis, Bifidobacterium longum* and *Bifidobacterium bifidum.*

8. Process according to any one of claims 1 to 7, **characterised in that**, at the end of the fermentation, the fermented product is dried.

9. Process according to claim 8, **characterised in that** the drying is carried out by atomisation.

10. Process according to claim 9, **characterised in that**, during the drying by atomisation, the exit air temperature is below 90°C.

11. Process according to claim 8, **characterised in that** the drying is carried out by lyophilisation.

12. Culture medium as defmed in any one of claims 1 to 3.

13. Process for preparing a fermented foodstuff enriched in galacto-oligosaccharides, **characterised in that** it comprises carrying out a process according to any one of claims 1 to 11.

14. Process according to claim 13, **characterised in that** it is carried out in order to prepare a fresh dairy foodstuff.

15. Process according to claim 14, **characterised in that** it is carried out in order to prepare a dehydrated dairy foodstuff having a β-galactosidase activity of between 0.5 and 5 U β-gal per gram of dry matter, and comprising between 0.5 and 5 g of galacto-oligosaccharides per 100 g of dry matter.

16. Process according to claim 15, **characterised in that** it is carried out in order to prepare a dehydrated dairy foodstuff having a β-galactosidase activity of between 1.5 and 3 U β-gal per gram of dry matter, and containing between 0.8 and 2.5 g of galacto-oligosaccharides per 100 g of dry matter.

17. Process according to any one of claims 15 and 16, **characterised in that** said dehydrated dairy foodstuff further comprises a lyophilisate of bifidobacteria.

18. Process according to claim 17, **characterised in that** said bifidobacteria are selected from among *Bifidobacterium breve, Bifidobacterium infantis, Bifidobacterium longum* and *Bifidobacterium bifidum.*

19. Process according to any one of claims 13 to 18, **characterised in that** said fermented foodstuff is intended as baby food.

## Patentansprüche

1. Verfahren zur Herstellung eines an Galactooligosacchariden und β-Galactosidase angereicherten fermentierten Produkts, wobei das Verfahren **dadurch gekennzeichnet ist, dass** es einen Schritt umfasst, bei dem man einen *Streptococcus thermophilus*-Stamm in einem Medium kultiviert, dessen Trockenmassekonzentration wenigstens 15 % (in Gewicht) beträgt, und das neben den für das Wachstum dieses Bakteriums notwendigen Nährstoffen wenigstens 0,1 % (in Gewicht Trockenmasse) eines Hydrolysats von Milchproteinen mit einem Hydrolysegrad zwischen 15 und 50 % und wenigstens 20 % (in Gewicht Trockenmasse) Lactose umfasst.

2. Verfahren nach Anspruch 1, **dadurch gekennzeichnet, dass** die Trockenmassekonzentration des Mediums zwischen 30 und 50 % (in Gewicht) liegt, und **dadurch**, **dass** das Medium zwischen 0,2 und 3 % (in Gewicht Trockenmasse) Hydrolysat von Milchproteinen und zwischen 25 und 60 % (in Gewicht Trockenmasse) Lactose umfasst.

3. Verfahren nach Anspruch 1, **dadurch gekennzeichnet, dass** das Hydrolysat von Milchproteinen einen Hydrolysegrad über 20 % hat.

4. Verfahren nach irgendeinem der Ansprüche 1 bis 3, **dadurch gekennzeichnet, dass** das Medium mit einem *S*. *thermophilus-*Stamm beimpft wird, der nach 4,5 h Kultivierung auf einem Medium gemäß Anspruch 3 in Kultur eine Aktivität über 0,9 U β-gal/g zeigt.

5. Verfahren nach Anspruch 4, **dadurch gekennzeichnet, dass** der *S*. *thermophilus-*Stamm ausgewählt ist aus der Gruppe bestehend aus:
- Stamm LFL-01, hinterlegt am 25. August 1994 bei der CNCM unter der Nummer I-1470;
- Stamm DN-001065, hinterlegt am 23. August 1995 bei der CNCM unter der Nummer I-1620.

6. Verfahren nach irgendeinem der Ansprüche 1 bis 5, **dadurch gekennzeichnet, dass** das Medium ferner mit Bifidobakterien inokuliert wird.

7. Verfahren nach Anspruch 6, **dadurch gekennzeichnet, dass** die Bifidobakterien ausgewählt sind aus der Gruppe bestehend aus *Bifidobacterium breve, Bifidobacterium infantis, Bifidobacterium longum und Bifidobacterium bifidum.*

8. Verfahren nach irgendeinem der Ansprüche 1 bis 7, **dadurch gekennzeichnet, dass** man das fermentierte Produkt nach Beendigung der Fermentation trocknet.

9. Verfahren nach Anspruch 8, **dadurch gekennzeichnet, dass** die Trocknung durch Zerstäuben erfolgt.

10. Verfahren nach Anspruch 9, **dadurch gekennzeichnet, dass** die Temperatur der Austrittsluft bei der Trocknung durch Zerstäuben unter 90 °C liegt.

11. Verfahren nach Anspruch 8, **dadurch gekennzeichnet, dass** die Trocknung durch Lyophilisation erfolgt.

12. Kulturmedium gemäß irgendeinem der Ansprüche 1 bis 3.

13. Verfahren zur Herstellung eines an Galactooligosacchariden angereicherten fermentierten Nahrungsmittels, **dadurch gekennzeichnet, dass** es die Durchführung eines Verfahrens nach irgendeinem der Ansprüche 1 bis 11 umfasst.

14. Verfahren nach Anspruch 13, **dadurch gekennzeichnet, dass** es zur Herstellung eines frischen Milchnahrungsmittels durchgeführt wird.

15. Verfahren nach Anspruch 14, **dadurch gekennzeichnet, dass** es zur Herstellung eines dehydratisierten Milchnahrungsmittels durchgeführt wird, das eine β-Galactosidase-Aktivität zwischen 0,5 und 5 U β-gal pro Gramm Trockenmasse besitzt und zwischen 0,5 und 5 g Galactooligosaccharide pro 100 g Trockenmasse umfasst.

16. Verfahren nach Anspruch 15, **dadurch gekennzeichnet, dass** es zur Herstellung eines dehydratisierten Milchnahrungsmittels durchgeführt wird, das eine β-Galactosidase-Aktivität zwischen 1,5 und 3 U β-gal pro Gramm Trockenmasse besitzt und zwischen 0,8 und 2,5 g Galactooligosaccharide pro 100 g Trockenmasse umfasst.

17. Verfahren nach irgendeinem der Ansprüche 15 oder 16, **dadurch gekennzeichnet, dass** das dehydratisierte Milchnahrungsmittel ferner ein Lyophilisat von Bifidobakterien umfasst.

18. Verfahren nach Anspruch 17, **dadurch gekennzeichnet, dass** die Bifidobakterien ausgewählt sind aus der Gruppe bestehend aus *Bifidobacterium breve, Bifidobacterium infantis, Bifidobacterium longum und Bifidobacterium bifidum.*

19. Verfahren nach irgendeinem der Ansprüche 13 bis 18, **dadurch gekennzeichnet, dass** das fermentierte Lebensmittel als Babynahrung vorgesehen ist.
